# EUROPEAN PATENT APPLICATION

(11) **EP 3 231 414 A2**
(43) Date of publication of application: **18.10.2017**
(21) Application number: 17162023.0
(22) Date of filing: 21.03.2017
(51) Int. Cl.: A61K 8/04, A61K 8/67, A61Q 17/04, A61Q 19/08, A61Q 19/00, A61K 8/31

(54) **SPRAY DISPENSER DEVICE AND COMPOSITION FOR TOPICAL USE COMPRISING A C14 OR C12 PLANT ALKANE**

(30) Priority: 21.03.2016 IT UA20161870
(71) Applicant: Ravetta, Cristiano, 27020 Torre d'Isola (Pavia) (IT); Facchini, Orfeo, 29020 Travo (Piacenza) (IT)
(72) Inventor: Ravetta, Cristiano, 27020 Torre d'Isola (Pavia) (IT); Facchini, Orfeo, 29020 Travo (Piacenza) (IT)
(74) Representative: Feltrinelli, Secondo Andrea

(57) **Abstract**

Dispenser device, for spray dispensing, comprising a container, a dispensing nozzle and a composition for topical use, adapted to be dispensed by means of spray, placed inside the container, wherein such composition comprises at least one active ingredient and an oily vehicle comprising a C14 or C12 alkane of plant derivation; composition that that can be sprayed or dispensed by means of spray or aerosol for topical use.

## Description

### TECHNICAL FIELD OF THE INVENTION

The present invention relates to a dispenser device for the spray dispensing of a composition for topical use and the composition for topical use that can be sprayed or adapted to be dispensed by means of spray dispensing.

In particular, such composition comprises liposoluble active ingredients dispersed in a natural oily vehicle, wherein such natural oily vehicle is of plant derivation, with high biodegradability index and low environmental impact.

### STATE OF THE PRIOR ART

Cosmetic or medical compositions for topical use are known which are present in spray bombs or other dispenser devices. Such known compositions, nevertheless, contain silicone compounds or cyclomethicone components or even volatile silicones, or synthetic products of non-plant derivation. However, silicone and/or synthetic compounds, even if they increase the diffusion on the skin of the product containing them, preventing the irritating greasy touch of other oily vehicles, have the disadvantage of having high environmental impact and over time being aggressive on the skin. Such composites, in fact, are in practice poorly biodegradable or, as with silicones, not at all biodegradable and cause skin irritations and dryness.

It is important to underline that modern dermatology is increasingly oriented towards the use of products certified by organisms that attest the natural-plant origin thereof, the complete harmlessness thereof, the absence of GMOs, the absence of residual solvents, the absence of substances harmful for man, such as derivatives from nanotechnologies, derivatives from animals (BSE), irradiated compounds or derivatives, and above all the eco-sustainability of the used raw materials.

It is well-known that lately the media has exerted strong pressures on the dermatological and cosmetic world for the substitution and consequent abolition of formulations containing palm oil and derivatives, in order to seek to reduce the considerable deforestation of forested areas and facilitate the replanting of oil palms.

In particular, silicones are used as emollients in creams and in other compositions. However, silicones have the defect of forming an impermeable film on the skin, which is not at all nourished by the cream or by the composition in question.

Indeed, for example by applying silicone creams, one obtains an increasingly dehydrated skin as a result.

Silicones are often also used for masking inferior quality formulations, lacking active ingredients: indeed, these give immediate gratification with the sensation of obtaining a soft skin, but the cosmetic substance is ineffective regarding skin nourishment and actually is damaging over time.

In addition, silicones are used for application on hair and confer aesthetic results with the first applications. After several applications, however, they tend to weigh down the hair, since they are washed away with difficulty, leaving a light impermeable film that increases over time.

From clinical tests, the lightest silicones (cyclomethicones) dry the skin where they are applied and are particularly contraindicated in case of skin that requires, indeed, hydration.

It has been proposed that there is actual sensitization to silicones.

Finally, silicones are not at all biodegradable and hence are not eco-compatible.

In addition, up to a low concentration (about 2% circa), silicones can be tolerated and their presence can improve the physical performances of spreadability of the cosmetic substances, but at overly high percentages (5% and upward), they are instead not skin-compatible and moreover cause serious ecological damage.

For such reason, the bodies set to control the level of pollution from chemical substances are considering banning the use of volatile silicones in cosmetic formulations by 2020.

### OBJECTS OF THE INVENTION

Therefore, the main object of the present invention is to improve the state of the art through the making of a spray dispenser device comprising a biocompatible composition.

Another object of the present invention is to provide a spray dispenser device comprising a composition that is 100% of plant origin or comprising ingredients of plant origin that are easily or completely biodegradable.

A further object of the present invention is to provide a composition for topical use, applicable on the skin and/or hair, lacking silicone compounds.

A still further object of the present invention is to provide a composition for topical use that is of plant derivation or which comprises ingredients of 100% plant origin, easily or completely biodegradable.

According to a first aspect of the present invention, a spray dispenser device is provided according to the enclosed claim 1.

According to a further aspect of the present invention, a composition for topical use is provided according to the enclosed claim 14.

Further advantageous or preferred characteristics of the present invention are described in the enclosed dependent claims.

### BRIEF DESCRIPTION OF THE DRAWINGS

Further characteristics and advantages of the present invention will be clearer from the detailed description of a preferred but not exclusive embodiment of a dispenser device according to the invention, illustrated by way of a non-limiting example in the enclosed set of drawings in which:
figure 1 illustrates a container of a dispenser device and a bag or BOV, before its insertion in the container itself,
figure 2 illustrates the container of figure 1 in which the bag or BOV is inserted and permanently fixed to the container itself,
figure 3 illustrates the container pursuant to figures 1 and 2, in which the composition according to the present invention is inserted in the bag or BOV,
figure 4 illustrates the dispenser device according to the present invention, completely assembled.

### EMBODIMENTS OF THE INVENTION

The present invention refers to a dispenser device.

Such dispenser device is of spray type.

According to one embodiment, such dispenser device comprises a spray bomb such as, for example, a BOV (Bag on Valve) bomb or a bomb with a no-gas dispenser or pump or a bomb made of aluminum or recyclable aluminum or plastic, or high-density plastic, or high-density polyethylene (HDPE).

Indeed, at least in one version of the invention, the oily vehicle could run the risk of oozing from a container made of plastic that is not high-density.

In one version of the invention, the dispenser device comprises a bomb whose relative container is not pressurized and does not contain chemical propellant gases. Such bomb is for example that sold with the name Airopack. A similar type of dispenser comprises a device for controlling the pressure and a container containing air, resulting in a controlled flow of the composition contained therein. This dispenser device can be made of plastic, e.g. recyclable plastic such as PET (polyethylene terephthalate).

Also this bomb type can be considered a BOV bomb.

Several advantages tied to the use of such containers are the following: the possibility of use of the container, during dispensing, in any position, i.e. also at 360°, the dispensing can be continuous and the product to be dispensed, as stated, is separated from the propellant. In addition, it is possible to dispense nearly all of the product contained in the BOV bomb, or better yet in its internal bag or container, and as will be better understood hereinbelow, the realization of such containers, such as the filling thereof, is very easy and quick.

The dispenser device comprises a container or package that delimits a space inside the container itself and a spray dispensing nozzle.

Such container comprises, at its interior, a composition for topical use, adapted to be dispensed by means of spray or sprayed, which will be better described hereinbelow.

The container has a lateral wall that delimits an internal surface and an external surface, the latter being adapted to be gripped by the user.

The container of the dispenser device has a substantially cylindrical shape, with circular, oval base, or parallelepiped shape, for example with a polygonal base, or other similar shapes.

The container of the dispenser device has one end closed by a bottom, adapted to be maintained during use in lower position; the other end, adapted during use to be maintained in upper position, is equipped with the dispensing nozzle. The internal space delimited by the container of the dispenser device can be completely isolated from the outside or not be airtight.

In the version in which the container of the dispenser device is airtight, this allows preserving the composition contained therein from oxidation.

Such composition can possibly be contained, within the container, in turn inside a casing or housing (such as in BOV bombs), so as to keep it separated from the surrounding environment within the container itself and hence prevent any type of alteration or oxidation.

The fact of preventing the composition contained in the dispenser device from being oxidized is of particular importance when such composition comprises one or more active ingredients which, once oxidized, reduce or lose their activity, such as vitamins or compounds of plant origin or derivation.

Such aspect is not to be considered obvious, since pump sprays (hence not airtight) exist on the market, which contain volatile silicone and vitamin E, but the latter can be oxidized. BOV and/or airtight bombs both have the property of being biocompatible and maintaining intact the properties of the active ingredients and the components contained in the formulation.

In addition, the dispenser device comprises means for actuating the dispensing of the composition according to the present invention, for example means actuatable by the user for the selective dispensing of the composition itself.

In one version of the invention, the space inside the container comprises a propellant fluid or gas capable of allowing the dispensing of the composition to be dispensed. The propellant fluid or gas, in one version of the invention, is compressed air, so as to be biodegradable.

In one version of the invention, in fact, the dispenser device does not comprise non-ecological propellant gases, in order to preserve the ecological nature of the present invention.

In a still further version of the invention, the dispenser device according to the present invention does not at all contain propellant gases.

The dispenser device can further comprise a valve, capable of allowing the dispensing of the composition once a specific pressure value has been exceeded.

The dispenser device according to the present invention, as is known, comprises a button, a key or a lever or a mechanism in general, actuatable by the user in order to cause the dispensing of the composition contained in the dispenser device.

Such composition is preferably isolated from the propellant fluid or gas.

In a further version of the invention, the dispenser device does not comprise a propellant fluid or gas but can comprise a dispensing pump.

The spray dispensing can occur in aerosol form.

Such dispenser device, according to a preferred version of the invention, is completely recyclable (100% recyclable) or in any case has low ecological or environmental impact.

The BOV bombs have a bag at their interior, for example made of laminar material, welded to an aerosol valve. The composition to be dispensed is injected inside the bag while the space inside the bomb (hence the space inside the container, between its internal wall and the bag) is filled with compressed air. By pressing the key of the dispenser, the compressed air pushes the product contained in the bag outward.

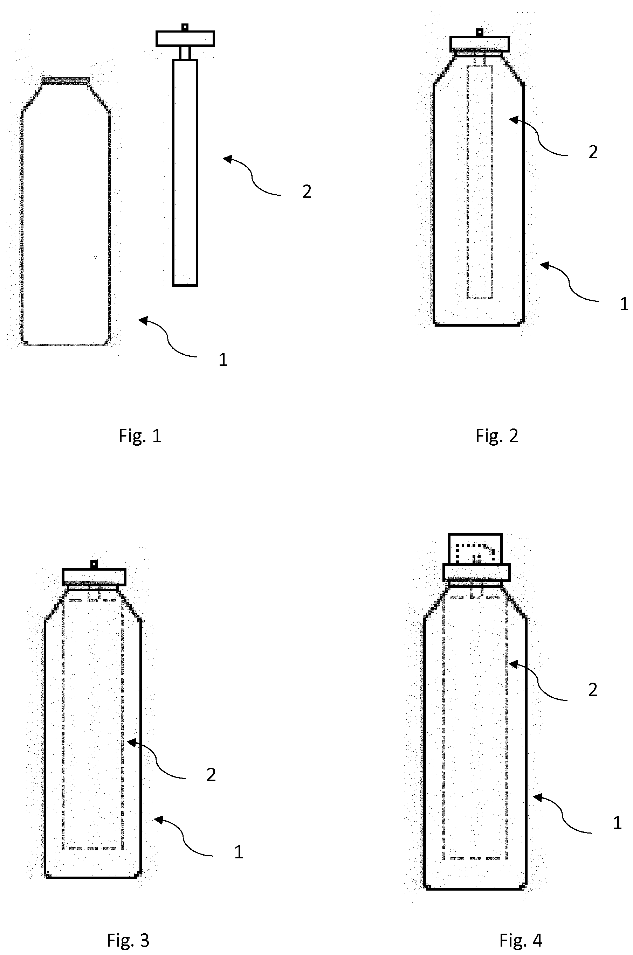

As illustrated in an exemplifying manner in the enclosed figures, therefore, the manner of production of such bombs comprises the following steps:
preparing the container 1 and inserting the bag or BOV 2 therein (figures 1 and 2),
placing the container 2 under pressure,
permanently fixing the bag or BOV to the container (figure 2),
inserting the product or the composition according to the present invention to be dispensed in the bag or BOV, which is consequently expanded (figure 3), completing the assembly and sealing the container 2 (figure 4).

The bombs with a no-gas dispenser or pump, which can be made of plastic that is 100% recyclable or completely recyclable, have the characteristic of functioning without the use of propellant gases. In addition, such bomb type is less expensive than the BOV bombs.

However, in using bombs with no-gas dispenser it is advisable that the composition to be dispensed contains a percentage of anti-oxidant, even if small, above all in the presence of liposoluble active ingredients that are sensitive to the oxygen in the air. Indeed, such bomb is not airtight.

BOV bombs, instead, being airtight, do not require compositions provided with anti-oxidant or preserving agents. In fact, being airtight, the composition at their interior maintains the characteristics thereof unaltered.

BOV bombs can be made, as stated, of recyclable aluminum. Due to this, such bombs are highly ecological.

Alternatively, the bombs according to the present invention or the BOV bombs can be made of plastic, or high-density plastic, or high-density polyethylene (HDPE). Also in such case, the plastic material can be recyclable. In such case, the bomb according to the present invention is highly ecological.

The dispenser device according to the invention comprises, at its interior, as stated, a composition for topical use.

In particular, such composition is adapted to be used for dermatological, dermocosmetic and/or trichological use.

Such composition can be sprayed or dispensed in spray or aerosol form.

Such composition has a strong hydrating, anti-oxidant, protective, nourishing, sebum-regulating and lenitive action, etcetera or at least one of such properties. The composition according to the present invention comprises at least one active ingredient, for example a first active ingredient.

The active ingredient according to the present invention comprises at least one liposoluble active ingredient.

The at least one active ingredient can have a nourishing, anti-oxidant, hydrating action for the skin and/or hair or at least one of such properties. Such active ingredient or first active ingredient can comprise, in one version of the invention, a liposoluble vitamin and/or Vitamin A and/or Vitamin E, in natural or synthetic form or in esterified form.

By Vitamin A, also the precursors can be intended, such as Beta Carotene in one version of the invention.

In one version of the invention, the first active ingredient comprises or consists of Vitamin E and/or Vitamin A and/or Vitamin E acetate and/or Vitamin A acetate.

The first active ingredient can be present in percentages comprised between 1% and 50% by weight over the total weight of the composition.

The at least one active ingredient can comprise at least one second active ingredient. The at least one active ingredient or the at least one second active ingredient comprises at least one from among the following components: short-chain PUFA (Polyunsaturated Fatty Acids), long-chain PUFA, a plant oil, for example a plant oil rich in PUFA, such as by way of example linseed oil, hempseed oil, kiwi seed oil, blackcurrent seed oil, Sacha Inchi seed oil, Moringa oil, Marula oil, passionflower seed oil, tamarind seed oil, a plant oil comprising or rich in liposoluble active ingredients such as alpha bisabolol, gamma oryzanol, or the liposoluble active ingredients themselves, etc., to be used as is or in a mixture.

The second active ingredient can be present in percentages comprised between 1% and 50% by weight over the total weight of the composition.

In one version of the invention, the active ingredient comprises a mixture of Vitamin A and/or Vitamin E and at least one second active ingredient. In such case, the active ingredient (considering the first and/or the second) can be present in percentages comprised between 1% and 50% by weight over the total weight of the composition.

The composition according to the present invention can comprise, in one version of the invention, a chemical or physical solar filter or an anti-UVA and/or anti-UVB solar filter.

In a preferred version of the invention, the at least one active ingredient and/or the at least one first and one second active ingredient are completely made of plant or are of plant derivation.

The present composition further comprises an oily vehicle.

Such oily vehicle is natural, in particular of plant derivation.

Such oily vehicle has high biodegradability and low environmental impact.

In a preferred version of the present invention, such oily vehicle is free of silicones or silicone derivatives; rather, in particular, such oily vehicle completely substitutes them.

The oily vehicle according to the present invention is completely made of plant or is of plant derivation.

The oily vehicle according to the present invention comprises at least one of the following plant components: oils of natural-plant derivation, C8 to C14 plant alkanes, used separately or mixed together, possibly in combination with at least one further liposoluble compound of plant or synthetic origin, such as an estolide, a polyglycerol ester, a plant oil, a plant oil rich in PUFA, plant squalane, propanediol dicaprylate, ethyl macadamiate, octyldodecyl olivate, hydrogenated ethylhexyl olivate, isodecyl neopentanoate, neopentyl glycol diheptanoate, diisopropyl sebacate, diisopropyl adipate, isododecane, an ester such as, only by way of example, isononyl isononanoate, isodecyl isononanoate, ethylhexyl isononanoate, methylheptyl isostearate, octyl dodecylcitrate crosspolymer, hexyldecanol, etcetera.

Since the oily vehicle is biocompatible and highly biodegradable, its components are preferably certified Ecocert, Cosmos and/or Natrue.

The oily vehicle can be present in percentages comprised between 50% and 99% by weight over the total weight of the composition.

The oily vehicle is liquid and/or the present composition is liquid.

Therefore, as can be observed, the composition according to the present invention, and/or in general the dispenser device comprising such composition, has highly ecological characteristics, due to the composition with high biodegradability index and low environmental impact and due to the package or container of the dispenser device itself that is completely recyclable.

In addition, the composition according to the present invention comprises ingredients that are highly active for the skin and/or hair and simultaneously are eco-compatible.

The possibly present liposoluble active ingredients are provided with high and effective dermatological and/or trichological activity, and simultaneously are ecological and biodegradable.

According to one version of the invention, the package or the container and/or the dispenser device is completely biodegradable and/or recyclable in all components thereof.

In such a manner, the present invention is 100% recyclable, with zero environmental impact and eco-sustainable with regard to the raw materials used (preventing deforestation, avoiding GMO cultivations and environmentally-damaging solvents such as chlorofluorocarbons or CFC, etc.). The use of liposoluble vitamins in dermatology, dermocosmetics and trichology has been well-known for many years. Vitamin E, for example, if correctly transmitted, is absorbed by the skin, has hydrating, anti-inflammatory and lenitive action. Moreover, Vitamin E, applied on the skin, reduces the formation of lipoperoxides and slows skin photoaging, in addition to acting as barrier against UV rays that are damaging for the skin.

Among the advantages of Vitamin A, we find - merely to give a few examples - anti-wrinkle action and improvement of collagen release, skin volumizing action and protection from the collagenase enzyme, which is activated by sunlight and which breaks the collagen.

PUFA are among other things capable of normalizing the intercellular lipid layers or they act as cementing substance. These lipids are responsible for the correct barrier functioning of the dermis. Still under discussion is the constitution of the barrier system: whether it is constituted by the entire corneal layer, whether it is limited to only the compact layer or whether it has its origins from the granular layer. The lipids of this compartment (ceramides and cholesterol) are organized as an osmotically active membrane that appears as lamellae localized in the intercellular spaces, occupying different and specific compartments; the diffusion through the corneal layer of the lipophilic and hydrophilic substances follows different paths. Over the last few years, various research studies have shown that the cells of the corneal layer are the seat of an intense biological activity, both intracellular and extracellular. PUFA are the main actors responsible for the correct functionality of the intercorneocyte cementing lipid substance (together with cholesterol and ceramides), probably due to the great capacity to fill the empty spaces between the cells of the corneal layer. Indeed, PUFA present the characteristic of having specific double bonds, with free carboxylic groups which form bridges of strong adherence due to the hydrogen ions of the carboxylic terminal, and due to this it is thought that they perform the indicated functions.

Nevertheless, PUFA are particularly delicate substances that tend to be subjected to the aggression of oxygen present in the air and of light, oxidizing and giving rise to a strong inactivity.

PUFA can also be present in the lipid matrix of many oils, which they enrich with their properties. Very often, unfortunately, these oils are also quite unstable, due in fact to the presence of PUFA which cause the oil to become rancid, causing an organoleptic alteration thereof and an alteration of dermatological activity. A classic example is linseed oil, highly useful from the dermatological and trichological standpoint, but practically unusable due to its high peroxidation index.

The use of the dispenser device according to the present invention allows reducing the drawbacks and the instability of the above-lamented components. The dispenser device according to the present invention, therefore, protects the abovementioned composites from light and from the oxygen present in the air, in particular when this is a spray bomb of BOV type.

Among the various proposed carriers, in one version of the invention, the compounds containing or based on plant alkanes base are comprised.

Such compounds have all the properties that make them excellent substitutes of the volatile silicones (e.g. the various cyclomethicones).

Such properties are at least one of the following: sensoriality, solubilizing force, evanescence, low density.

Indeed, such properties render the compounds comprising or based on plant alkane very similar to the silicones normally used, regarding the appearance that is perceived on the skin or hair when they are applied.

For example, the compounds containing or based on plant alkane have a feel on the skin and an evanescent effect similar to that of the known silicone compounds, such as the volatile silicones. In addition, to the contrary of the latter, the compounds containing or based on plant alkane have proven to be more eudermic, less dehydrating for the skin, in addition to being completely biodegradable.

In one version of the invention, the oily vehicle comprises (or in a further version of the invention consists of) a C14 plant alkane or a C14 plant alkane in combination with a further liposoluble compound.

The further liposoluble compound is preferably completely vegetal or of plant derivation.

In a further version of the invention, the oily vehicle comprises (or in a further version of the invention consists of) a C12 plant alkane, or a C12 plant alkane in combination with a further liposoluble compound.

In a preferred version of the invention, the C12 alkane in combination with a further liposoluble compound is a C12 alkane mixed with an estolide, in particular with a plant estolide, such as an estolide derived from olive oil.

In a preferred version of the invention, the C14 alkane in combination with a further liposoluble compound is a C14 alkane mixed with an estolide, in particular with a plant estolide, such as an estolide derived from olive oil. Such compound is glyceryl olivate oleate estolide and tetradecane or glyceryl olivate oleate estolide and dodecane.

In a preferred version of the invention, the C14 alkane or C12 alkane in combination with a further liposoluble compound is a C14 alkane or C12 alkane with hexyldecanol.

Preferably the plant alkane is derived from the oil of Brassica Campestris (kale) or from Brassica Napus (rapeseed). In particular, the plant alkane is a product that is 100% biodegradable, of derivation, beyond that vegetal, also eco-sustainable. Indeed, such plant alkanes are not derived from palm oil, since the use thereof is not ecologically compatible.

In one version of the invention, the above oily vehicle is the only oily component of the composition.

The mixture comprising a C14 plant alkane and an olive oil estolide comprises 1-99% of C14 plant alkane and 99-1% of olive oil estolide, the percentages being weight/weight over the total of this mixture or of the oily vehicle, in one version of the invention.

The mixture comprising a C12 plant alkane and an olive oil estolide comprises 1-99% of C12 plant alkane and 99-1% of olive oil estolide, the percentages being weight/weight over the total of this mixture or of the oily vehicle, in one version of the invention.

In a still further version of the invention, the mixture comprises a C14 or C12 plant alkane and an oily vehicle comprises 1-99% of C14 plant alkane or C12 plant alkane and 99-1% of estolide from olive oil and/or hexyldecanol, the percentages being weight/weight over the total of this mixture or of the oily vehicle.

Such compound, at least in one version of the invention, is particularly preferred since it has an optimal eudermicity, an optimal carrier power for the vitamins or other active ingredients described above, improving the skin penetration and, therefore, the activity. In addition, it is completely innocuous on skin and/or on hair, not irritating for the eyes, and completely biodegradable.

A further advantage of the oily vehicle according to the present invention, for example in particular of the compounds based on plant alkane or of a C14 or C12 plant alkane with an estolide, for example derived from olive oil, and/or with plant hexyldecanol is that of not blocking the spray dispensing or not obstructing the relative nozzle thereof. This aspect is particularly important when the dispenser device is a spray bomb BOV, and when the oily vehicle is present in the composition with high concentrations of vitamins and/or plant oils and/or other lipids or active ingredients.

Therefore, even after prolonged use, there is no blocking of the dispensing, nor damaging of the casing inside the bomb.

In addition, the present invention maintains unaltered over time the active characteristics of products mixed in the above-described oily vehicle, which is not oxidized nor does it allow the oxidation of the composition in which it is contained.

The oily vehicle and/or the composition according to the present invention, on the skin and/or hair, confers a sensation of dryness, i.e. not greasy and evanescent, hence it leaves a talc feel.

In one version of the invention, the oily vehicle and/or the present composition is colorless and without odor; according to other versions, fragrances or deodorant or fragrant substances can be added.

Hereinbelow, several examples of the present invention will be reported, only by way of a non-limiting example of the composition according to the present invention.

In particular, such composition is applied by means of spray, for example by using a dispenser device of BOV spray bomb type.

The reported percentages are indicated weight/weight over the total of the composition.

According to a preferred version, the following examples provide that the oily vehicle is a plant alkane mixed with a further liposoluble compound such as a plant estolide or, still more preferably, a C14 or C12 plant alkane mixed with an olive oil estolide or still more preferably a C14 or C12 plant alkane mixed with a hexyldecanol.

**Composition 1**

| | |
|---|---|
| Vitamin E acetate | 20% |
| Oily vehicle | 80% |

**Composition 2**

| | |
|---|---|
| Natural Vitamin E | 20% |
| Oily vehicle | 80% |

**Composition 3**

| | |
|---|---|
| Vitamin A acetate | 20% |
| Oily vehicle | 80% |

**Composition 4**

| | |
|---|---|
| Beta carotene | 10% |
| Oily vehicle | 90% |

**Composition 5**

| | |
|---|---|
| PUFA | 10% |
| Oily vehicle | 90% |

**Composition 6**

| | |
|---|---|
| Plant oils titrated in PUFA | 30% |
| Oily vehicle | 70% |

**Composition 7**

| | |
|---|---|
| Vitamin E acetate | 20% |
| Solar filters UVA-UVB | 10% |
| Oily vehicle | 70% |

**Composition 8**

| | |
|---|---|
| Natural Vitamin E | 20% |
| Solar filters UVA-UVB | 10% |
| Oily vehicle | 70% |

**Composition 9**

| | |
|---|---|
| Natural Vitamin E | 20% |
| Alpha Bisabolol | 1% |
| Oily vehicle | 79% |

In one version, the composition according to the invention consists of at least one active ingredient and one oily vehicle, wherein the at least one active ingredient comprises at least one first active ingredient and/or at least one second active ingredient while the oily vehicle comprises (or consists of) a C14 alkane or a C14 alkane in combination with a further liposoluble compound or a C12 alkane or a C12 alkane in combination with a further liposoluble compound.

In one version of the invention, the composition comprises 10% to 30% of active ingredient by weight over the total weight of the composition and 70% to 90% of oily vehicle by weight over the total weight of the composition.

In one version of the invention, the composition and/or the oily vehicle do not contain silicones, cyclosilicones or volatile silicones.

In one version of the invention, beyond the abovementioned oily vehicle, hence possibly containing plant hexyldecanol or a plant alkane with hexyldecanol, the composition according to the present invention can comprise alpha bisabolol as well as, for example, Vitamin E or Vitamin E acetate.

In at least one version of the invention, therefore, alpha bisabolol constitutes the at least one second active ingredient, considering that the at least one first active ingredient comprises or is constituted by a vitamin, such as vitamin E, in simple or acetate form.

In one version of the invention, the composition according to the present invention comprises a plant alkane as oily vehicle, vitamin E as first active ingredient and, as second active ingredient, alpha bisabolol.

It is thus seen that the invention allows providing, in dermatological, dermocosmetic and/or trichological formulas, a highly active compound, conveyed with an oily vehicle or a light lipid of plant derivation, in spray form. In such a manner, it is possible to overcome the previously lamented problems of the prior art, in which volatile silicones are used, by providing very active products that are conveyed with an oily vehicle that leaves the functions thereof unaltered, that accentuates the biodegradability thereof, and hence the eco-sustainability and the possibility to be obtained from renewable sources.

In addition, the sprayable composition according to the present invention, in at least one version thereof, is very simple and natural, comprising a limited number of components and reducing the presence of synthetic compounds to the minimum, given that it is as much as possible natural or vegetal or deriving from renewable plant sources.

The present composition is a nourishing and/or anti-oxidant and/or hydrating composition for skin and/or hair, simultaneously able to transmit the active ingredient contained therein and have it be absorbed by the skin or by the hair themselves.

All these advantages are obtained with the present invention, which at least in one version thereof provides for both the active ingredients and the oily vehicle derived from plant origins and/or from renewable sources and capable of conferring the same or improved characteristics, when applied and as application mode, with respect to the synthetic compounds of known type, such as silicone compounds.

The present invention has been described according to preferred embodiments, but equivalent variants can be conceived without departing from the protective scope offered by the following claims.

## Claims

1. Dispenser device, for spray dispensing, comprising a container, a dispensing nozzle and a composition for topical use, adapted to be dispensed by means of spray, placed inside said container, wherein said composition comprises at least one active ingredient and one oily vehicle, **characterized in that** said at least one active ingredient comprises at least one first active ingredient and/or at least one second active ingredient and **in that** said oily vehicle is vegetal or of plant derivation and comprises a C14 alkane of plant derivation or a C14 alkane of plant derivation in combination with a further liposoluble compound or a C12 alkane of plant derivation or a C12 alkane of plant derivation in combination with a further liposoluble compound.

2. Dispenser device according to claim 1, wherein said dispenser device comprises a spray bomb such as a BOV (Bag on Valve) bomb or a bomb with a no-gas dispenser or pump and/or a bomb made of aluminum, recyclable aluminum or plastic, or high-density plastic, or high-density polyethylene (HDPE) and/or wherein said container is airtight so as to preserve said composition from oxidation and/or wherein said composition is contained, within said container, inside a casing or housing, in a manner so as to keep it separate from the surrounding environment inside the container itself and/or wherein said composition is contained in a casing or housing, placed inside said container, in a manner so as to prevent the oxidation of the composition itself and/or wherein said container is airtight.

3. Dispenser device according to claim 1 or 2, wherein said at least one active ingredient has a nourishing, anti-oxidant, hydrating action for the skin and/or hair, dermatological and/or trichological activity or at least one of such properties and/or wherein said composition is a cosmetic or dermatological or trichological or medical composition and/or wherein said at least one active ingredient is liposoluble and/or wherein said at least one first active ingredient comprises a liposoluble vitamin and/or Vitamin A and/or Vitamin E, in natural or synthetic form or in esterified form and/or a precursor of Vitamin A such as Beta Carotene.

4. Dispenser device according to any one of the preceding claims, wherein said at least one second active ingredient comprises at least one from among the following components: short-chain PUFA, long-chain PUFA, a plant oil, a plant oil rich in PUFA, such as linseed oil, hempseed oil, kiwi seed oil, blackcurrent seed oil, Sacha Inchi seed oil, Moringa oil, Marula oil, passionflower seed oil, tamarind seed oil, a plant oil comprising liposoluble active agents such as alpha bisabolol, gamma oryzanol, or the liposoluble active ingredients themselves, etcetera.

5. Dispenser device according to any one of the preceding claims, wherein said first active ingredient comprises or consists of Vitamin E acetate and/or Vitamin A acetate and/or alpha bisabolol, and/or wherein said first active ingredient is present in a percentage comprised between 1% and 50% by weight over the total weight of the composition.

6. Dispenser device according to any one of the preceding claims, wherein said second active ingredient is present in a percentage comprised between 1% and 50% by weight over the total weight of the composition and/or wherein said first active ingredient and said second active ingredient are present overall in a percentage comprised between 1% and 50% by weight over the total weight of the composition.

7. Dispenser device according to any one of the preceding claims, wherein said oily vehicle is present in a percentage comprised between 50% and 99% or between 70% and 90% by weight over the total weight of the composition.

8. Dispenser device according to any one of the preceding claims, wherein said composition consists of said at least one active ingredient, and/or in said at least one second active ingredient, and in said oily vehicle.

9. Dispenser device according to any one of the preceding claims 1 to 7, wherein said composition comprises a chemical or physical solar filter or an anti-UVA and/or anti-UVB solar filter and/or wherein said composition and/or said oily vehicle do not contain silicones, cyclosilicones or volatile silicones.

10. Dispenser device according to any one of the preceding claims, wherein said composition contained therein comprises:
20% by weight of Vitamin E acetate or natural Vitamin E and 80% by weight of said oily vehicle or
20% by weight of Vitamin A acetate and 80% by weight of said oily vehicle or
10% by weight of Beta Carotene and 90% by weight of said oily vehicle or
10% by weight of PUFA and 90% by weight of said oily vehicle or
30% by weight of plant oils titrated in PUFA and 70% by weight of said oily vehicle or
20% by weight of Vitamin E acetate or natural Vitamin E, 10% by weight of at least one UVA-UVB solar filter and 70% by weight of said oily vehicle, or
20% by weight of Vitamin E acetate or natural Vitamin E, 1% by weight of at least one active plant ingredient such as alpha bisabolol and 79% by weight of said oily vehicle.

11. Dispenser device according to any one of the preceding claims, wherein said oily vehicle and/or said at least one further liposoluble compound of plant origin comprises at least one of the following components: an estolide, a polyglycerol ester, a plant oil, a plant oil rich in PUFA, plant squalane, propanediol dicaprylate, ethyl macadamiate, octyldodecyl olivate, hydrogenated ethylhexyl olivate, isodecyl neopentanoate, neopentyl glycol diheptanoate, diisopropyl sebacate, diisopropyl adipate, isododecane, an ester such as isononyl isononanoate, isodecyl isononanoate, ethylhexyl isononanoate, methylheptyl isostearate, octyl dodecylcitrate crosspolymer, hexyldecanol, etcetera.

12. Dispenser device according to claim 1, wherein said C14 or C12 alkane in combination with a further liposoluble compound is a C14 or C12 alkane mixed with an estolide or with an estolide derived from a plant oil or with an estolide derived from olive oil or with hexyldecanol and/or wherein said C12 alkane or C14 in combination with a further liposoluble compound is glyceryl olivate oleate estolide and dodecane or tetradecane.

13. Dispenser device according to any one of the preceding claims, wherein said dispenser device and/or said composition are biodegradable or derived from plant sources or from renewable sources.

14. Composition, adapted for topical applications on the skin or hair or for topical use, comprising at least one active ingredient and an oily vehicle, wherein said at least one active ingredient comprises at least one first active ingredient and/or at least one second active ingredient and wherein said oily vehicle is vegetal or of plant derivation and comprises a C14 alkane of plant derivation or a C14 alkane of plant derivation in combination with a further liposoluble compound or a C12 alkane of plant derivation or a C12 alkane of plant derivation in combination with a further liposoluble compound, wherein said composition can be dispensed in spray or aerosol form by means of a spray dispenser device, according to any one of claims 1 to 13.

15. Use of the dispenser device according to one or more of claims 1 to 13 for dispensing a composition according to the preceding claim, wherein said use is for cosmetic applications.

16. Composition comprising at least one active ingredient and an oily vehicle, wherein said at least one active ingredient comprises at least one first active ingredient and/or at least one second active ingredient and wherein said oily vehicle is vegetal or of plant derivation and comprises a C14 alkane of plant derivation or a C14 alkane of plant derivation in combination with a further liposoluble compound or a C12 alkane of plant derivation or a C12 alkane of plant derivation in combination with a further liposoluble compound according to any one of the claims 1 to 13, wherein said composition can be dispensed in spray or aerosol form by means of a spray dispenser device, wherein said composition is for use in the dermatological or trichological or medical topical treatment of pathologies or dysfunctions or disturbances of the skin or hair.
